(19) Europäisches Patentamt
European Patent Office
Office européen des brevets

(11) Publication number: 0 484 547 A1

(12) # EUROPEAN PATENT APPLICATION published in accordance with Art. 158(3) EPC

(21) Application number: 91909097.7

(51) Int. Cl.$^5$: A61B 5/02, A61B 5/14

(22) Date of filing: 17.05.91

(86) International application number: PCT/JP91/00658

(87) International publication number: WO 91/17700 (28.11.91 91/27)

(30) Priority: 18.05.90 JP 128823/90

(43) Date of publication of application: 13.05.92 Bulletin 92/20

(84) Designated Contracting States: BE CH DE FR GB LI NL SE

(71) Applicant: MITSUBISHI RAYON CO., LTD.
3-19, Kyobashi 2-chome Chuo-Ku
Tokyo 104(JP)

(72) Inventor: IWASAKI, Hitoshi,
Nukazukashukusha 3-402
42, Azanukazuka Yanagida Akita-shi
Akita 010(JP)

(74) Representative: Hansen, Bernd, Dr. Dipl.-Chem.
Hoffmann, Eitle & Partner Patent- und Rechtsanwälte Arabellastrasse 4 Postfach 81 04 20
W-8000 München 81(DE)

(54) **CATHETER FOR MEASUREMENT AND METHOD OF MEASURING OXYGEN SATURATION DEGREE OR FLOW SPEED OF BLOOD.**

(57) A catheter for measurement comprising light emitting and light receiving optical fibers inserted into the catheter in which respective tip surfaces of both optical fibers are disposed face-to-face and apart from each other at a required distance on the outer wall of the catheter and fixed thereto. Rays of light for measurement and reference are emitted from the light emitting fiber of the catheter and, by measuring the degree of penetration of said two kinds of light emitting from the light receiving optical fiber after passing through blood present between the tip surfaces of both optical fibers, an oxygen saturation degree or flowing speed of blood can be found in real time.

FIG. 1

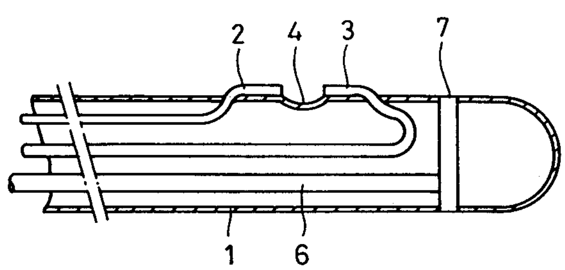


EP 0 484 547 A1

## Technical Field

This invention relates to an intravascularly retained catheter for measuring the degree of oxygen saturation and flow rate of the blood circulating through human blood vessels and the like on a real-time basis. It also relates to a method for measuring the degree of oxygen saturation and flow rate of blood by using such a catheter wherein higly accurate measurements can be made even in the presence of various disturbing factors.

## Background Art

In order to measure the degree of oxygen saturation of the blood circulating through blood vessels on a real-time basis, there has been known an oxygen saturation measuring catheter of light reflection type which utilizes the light reflected from the bood. Specifically, this catheter has a pair of optical fibers disposed parallel to each other. The end faces of these optical fibers are exposed at the front end of the catheter, one of the optical fibers being used to emit the measuring light and the other being used to receive the reflected light. The light emerging from the light-emitting optical fiber is reflected by red blood cells and the reflected light is collected by the light-receiving optical fiber. Then, the degree of oxygen saturation can be determined from the reflected light.

However, this oxygen saturation measuring catheter of light reflection type has the following disadvantages.

(1) If the tip of the catheter is in proximity to or in contact with the blood vessel wall, the light reflected from the blood vessel wall, and not from the red blood cells, is detected. This leads to large measuring errors.

(2) Calculation of the degeee of oxygen saturation is based on the linear relationship between the degree of oxygen saturation and the reflected light intensity ratio. However, variations in blood pH, hemoglobin concentration, blood flow rate and the like tend to disturb the linearity, so that it becomes difficult to make highly accurate measurements with good reproducibility.

Such a catheter has been considered to be usable for the sole purpose of measuring the degree of oxygen saturation of the mixed venous blood flowing through a pulmonary artery, because contact of the catheter tip with the blood vessel wall can be avoided.

On the other hand, the temperature dilution method using a thermistor has been known as a method for measuring the flow rate of the blood circulating through blood vessels. According to this method, blood flow velocity is measured by attaching a thermistor to a catheter, injecting distilled water or physiological saline having a temperature lower than that of the blood, and monitoring its temperature changes with the thermistor. This method has the advantage that the marker substance is dispersed and not recirculated. However, the above-described temperature dilution method has the following disadvantages.

(1) Since slight temperature changes of the order of 1 to 2°C are measured in most cases, the signal-to-noise ratio is small and the measurement is affected by fluctuations of the base line.

(2) Since heat is lost before injection and during injection, the temperature of the injected fluid must be measured in the vicinity of the injection orifice.

(3) Where the catheter is inserted into a blood vessel in the direction opposite to the flow of blood, the heat transfer within the catheter produces error in the measurement of blood flow rate.

As described above, the oxygen saturation measuring catheter of light reflection type has a structure which makes it difficult to apply it to blood vessels other than the pulmonary arteries, and the temperature dilution method using a thermistor has the disadvantage of involving many factors which produce errors in the measurement of blood flow rate.

## Disclosure of the Invention

It is an object of the present invention to provide a measuring catheter which enables the degree of oxygen saturation and flow velocity of the blood circulating through blood vessels to be measured in real time with good reproducibility and with high accuracy.

Another object of the present invention is to provide a method for measuring the degree of oxygen saturation and flow rate of the blood circulating through blood vessels in which measurements can be made with high accuracy even in the presence of various disturbing factors.

According to one aspect of the present invention, there is provided a measuring catheter having light-emitting and light-receiving optical fibers disposed within the catheter, the end faces of the light-emitting and light-receiving optical fibers being fixed to the outer sidewall of the catheter so that they are opposite to each other with a predetermined space therebetween.

According to another aspect of the present invention, there is provided a method for measuring the degree of oxygen saturation of blood which comprises providing the above-described catheter into a stream of blood, supplying light having a wavelength of about 805 nm and visible light to the light-emitting optical fiber, either simultaneously or alternately, measuring the transmittances of the two types of light that are collected by the light-receiving optical fiber after having passed through the blood present in the gap between the end faces of both optical fibers, and determining the degree of oxygen saturation of the blood on the basis of the transmittances of the two types of light.

According to still another aspect of the present invention, there is provided a method for measuring blood flow rate which comprises providing the above-described catheter into a stream of blood, supplying light having a wavelength of about 805 nm and visible light or light having a wavelength of 900 nm or longer to the light-emitting optical fiber, either simultaneously or alternately, measuring the transmittances of the two types of light that are collected by the light-receiving optical fiber after having passed through the marker substance-containing blood present in the gap between the end faces of both optical fibers, and determining the blood flow velocity on the basis of the transmittances of the two types of light.

When the measuring catheter of the present invention is used, the light is absorbed by the hemoglobin contained in the red blood cells present in the gap between the end faces of the light-emitting and light-receiving optical fibers, i.e. the volume (measuring zone) as defined by the length of the gap and the cross-sectional area of the optical fibers. Accordingly, by using two types of light having selected wavelengths, the degree of oxygen saturation can be determined from the ratio between the transmitted light intensities at these wavelengths. Moreover, it has been demonstrated that the degree of oxygen saturation can be measured with high accuracy even if the optical fibers are in contact with the blood vessel wall. Furthermore, blood flow rate can also be measured by injecting a solution of a specific dye into the blood, for example, from the tip of the catheter, and monitoring changes in transmitted light intensity when the dye passes through the gas (measuring zone) between the end faces of the optical fibers.

## Brief Description of the Drawings

Fig. 1 is a schematic sectional view of a measuring catheter in accordance with the present invention;

Fig. 2 is a schematic sectional view illustrating another embodiment of the emasuring catheter of the present invention which is provided with a lumen for use in blood sampling;

Fig. 3 is a schematic diagram illustrating the construction of an oxygen saturation measuring apparatus using the measuring catheter of the present invention;

Fig. 4 is a graph illustrating the light absorption curves of hemoglobin and a marker substance;

Fig. 5 is a schematic view illustrating a testing apparatus for altering the degree of oxygen saturation of blood and measuring it;

Fig. 6 is a schematic view illustrating a testing apparatus for altering blood flow rate and measuring it;

Fig. 7 is a graph in which the difference between the transmittances (logarithmically converted values) of two wavelengths of light is plotted against the degree of oxygen saturation;

Fig. 8 is a graph showing variations in output values when the color of the inner wall of the tube for letting flow the blood to be measured was changed from white to black;

Fig. 9 is a graph showing variations in output values when the pH of the blood was altered;

Fig. 10 is a graph showing the relationship between the values obtained by measurement according to the method of the present invention and those obtained by measurement with a commercially available Hemoximeter;

Fig. 11 is a graph showing the variation of transmittance at 805 nm with Indocyanine Green (ICG) concentration; and

Fig. 12 is a graph showing the relationship between the values obtained by measurement according to the method of the present invention and those obtained by direct measurement, in which solid circles represent the measured values for water and open circles represent those for blood.

## Best Mode for Carrying Out the Invention

The present invention is more specifically described hereinbelow with reference to the accompanying drawings.

First of all, the selection of wavelengths of light for use in the present invention is explained. Fig. 4 is a graph showing the variation in absorbance between oxyhemoglobin and reduced hemoglobin. As can be seen from this graph, large differences in absorbance are observed in the visible region, but there is no difference in absorbance at a wavelength of about 805 nm. Accordingly, if the reference light is selected to have a wavelength of 805 nm and the measuring light is selected to have a wavelength in the visible region (e.g., in the range of 500 to 760 nm), measurements can be made without undergoing the influence of various disturbing factors as described previously. Moreover, the use of these two types of light, coupled with the employment of transmission type construction, also makes it possible to make measurements without undergoing the influence of the blood vessel wall and the like.

More specifically, the employment of transmission type construction minimizes the possibility of picking up the light reflected from the blood vessel wall. Moreover, although the transmitted light intensities at the measuring and reference wavelengths may vary under the influence of various disturbing factors, the ratio of the transmitted light intensity at the measuring wavelength to that at the reference wavelength remains constant even in the presence of various disturbing factors, provided that the hemoglobin concentration is constant. For these reasons, the degree of oxygen saturation and flow rate of the blood can be measured with good reproducibility.

In the present invention, there is used a catheter having light-emitting and light-receiving optical fibers whose end faces are fixed to the outer sidewall of the catheter so that they are opposite to each other with a predetermined space therebetween. When this catheter is inserted into a stream of blood, the light emerging from the end face of the light-emitting optical fiber passes through the blood and falls on the end face of the light-receiving optical fiber. Thus, measurements are made by monitoring the intensity of the transmitted. In order to avoid measuring errors due to the stagnation of blood between the end faces of the optical fibers and the resulting failure of fresh blood supply, the distance between the end faces should preferably be sufficiently greater than the diameter of the optical fibers. However, if the distance between the end faces is too great, the intensity of the transmitted light will become excessively low, thus causing a reduction in measuring accuracy. The optical fibers preferably have a diameter of 70 to 300 $\mu$m, because they can be bent with a sufficiently small radius of curvature and they can transmit the required quantity of light.

When the hemoglobin concentration and the measuring wavelength are taken into consideration on the basis of the relationship between the transmitted light and the scattered light, the optimum distance between the end faces for measuring the degree of oxygen saturation of blood is about 1.0 mm at a hemoglobin concentration of 10 mmol/l (16.1 g/l) and a measuring wavelength of 670 nm. Accordingly, when the range of variation of hemoglobin concentration in the human body, the region of the measuring wavelength, the above-described relationship of the distance between the end faces and the diameter of the optical fibers, and the preferred diameter of the optical fibers are taken into consideration, the distance between the end faces is preferably in the range of 0.75 to 1.5 mm.

In order to measure blood flow rate, the same catheter as described above may be provided with a marker substance transfer tube whose outlet end is formed at a position which permits a marker substance (dye) to mix with the blood intimately and flow through the gap between the end faces of the two optical fibers. This makes it possible to employ the dye dilution method in which measurements are made by using a wavelength of light highly absorbable by the marker substance and another wavelength of light less absorbable thereby while injecting the marker substance into the blood. For example, Indocyanine Green can be used as the marker substance. Indocyanine Green exhibits maximum absorption at a wavelength of 805 nm and less absorption at wavelengths longer than 870 nm or shorter than 680 nm. Accordingly, blood flow rate can be measured by using light having a wavelength of about 805 nm and light having a wavelength of, for example, about 900 nm or 670 nm.

Fig. 1 is a sectional view of a measuring catheter in accordance with the present invention with which measurements are made by sending out light into the blood from the end face of one optical fiber and collecting the light having passed through the blood by the end face of the other optical fiber. A light-emitting optical fiber 2 and a light-receiving optical fiber 3 are disposed within a catheter 1 designed to be retained intravascularly, and the end faces of optical fibers 2 and 3 are fixed to the outer sidewall of catheter 1 so that they are opposite to each other with a predetermined space, preferably in the range of 0.75 to 1.5 mm, therebetween. The same purpose can also be accomplished by embedding optical fibers 2 and 3 in the sidewall of catheter 1 and fixing their end faces so that they are opposite to each other with a predetermined space therebetween. Moreover, optical fibers 2 and 3 may used as light-receiving and light-emitting optical fibers, respectively. Furthermore, although the end faces of optical fibers 2 and 3 are oppositely disposed along the longitudinal axis of catheter 1 in the embodiment illustrated in Fig. 1, they can also be oppositely disposed so as to traverse the longitudinal axis. In order to facilitate the removal of a blood clot, a recess 4 is formed in the sidewall of catheter 1 between the end faces of optical fibers 2 and 3.

As illustrated in Fig. 3, the other ends of optical fibers 2 and 3 are connected to light sources and photodetectors by way of a photoconnector 5. Moreover, the outlet end of marker substance transfer tube 6 disposed within catheter 1 for the purpose of measuring blood flow rate is opened in the sidwall of catheter 1. A plurality of outlets 7 are formed along the circumference of catheter 1 so that the marker substance may be mixed with the blood intimately. The inlet end of transfer tube 6 is connected to a liquid feeding device 8 as illustrated in Fig. 3. In Fig. 3, catheter 1 is inserted into a blood vessel 9 in the direction opposite to the flow of blood as shown by an arrow, and the outlet end of transfer tube 6 is opened in the sidewall of catheter 1 at positions close to its tip. However, where catheter 1 is inserted in the same direction as the flow of blood, the outlet end of transfer tube 6 is opened in the sidwall of catheter 1 at positions remote from its tip.

If a marker substance (dye) can be supplied so as to mix with the blood intimately and flow through the gap between the end faces of the optical fibers, for example, by using another catheter for supplying a marker substance, the catheter of the present invention need not be provided with the transfer tube. In such a case, the catheter may be constructed as illustrated in Fig. 2.

In the embodiment illustrated in Fig. 2, light-transmitting and light-receiving optical fibers are disposed within a catheter having a lumen 15 for use in blood sampling, and the end faces of both optical fibers are fixed to the outer sidewall of the catheter so that they are opposite to each other with a predetermined space therebetween.

Now, the method for the degree of oxygen saturation and flow rate of blood by using the catheter of the present invention is described with reference to Fig. 3.

Light form a laser diode 10 having an emission wavelength of 670 nm and light from a laser diode 10' having an emission wavelength of 805 nm are combined by means of an optical divider 11, and the combined light is supplied to light-emitting optical fiber 2 by way of an optical connector 5. The light having passed through the blood is collected by light-receiving optical fiber 3, conducted to an optical divider 11' by way of optical connector 5, and divided into two parts. One part of the light is fed to a photodetector 13 by way of a dichroic mirror 12 which transmits light at 670 nm, and the other part is fed to a photodetector 13' by way of a dichroic mirror 12' which transmits light at 805 nm. The outputs (i.e., transmittances at 670 nm and 805 nm) obtained from photodetectors 13 and 13' are processed in an arithmetic unit 14 to calculate the ratio between the transmittances.

The above-described embodiment includes an optical divider for use on the light emission side, an optical divider for use on the light reception side, and two sets of dichroic mirrors and photodetectors. Alternatively, it is also possible to use one set of dichroic mirror and photodetector on the light reception side, supply two types of light alternately, and measure the transmittances thereof synchronously.

According to the above-described measuring method, laser light having two different wavelengths is made to fall on the red blood cells present in the gap between the opposite end faces of optical fibers 2 and 3 of catheter 1 retained in blood vessel 9. Then, the degree of oxygen saturation of the blood can be determined by spectroscopically analyzing the transmitted light which has been partially absorbed by the hemoglobin contained in the red blood cells. Moreover, the blood flow rate can also be measured by injecting a marker substance from the openings of transfer tube 6 into the blood so that it passes through the gap between the end faces of both optical fibers.

The present invention is further illustrated by the following example.

## Example

Rabbit blood which had been collected within an hour was diluted with Ringer's solution to prepare blood samples having a hemoglobin concentration in the range of 1.0 to 16.0 g/dl. Using the apparatus of Fig. 5 including a disk oxygenator 16 for gas exchange, the degree of oxygenation saturation of a blood sample was altered by varying the nitrogen/oxygen ratio of the influent gas while keeping the carbon dioxide concentration at 5% to minimize variation in blood pH. Using a silicone rubber tube 17 in place of a blood vessel, the blood sample was circulated through silicone rubber tube 17 with the aid of a pump so as to pass through a heat exchanger 19 and pass by a catheter 18 and a thermocouple 20. The blood flow rate was controlled by pump 21. The measuring catheter used had the structure illustrated in Fig. 1 and the distance between the end faces of both optical fibers was 12 mm.

Separately, the pH, $pO_2$ and $pCO_2$ of the blood sample were measured with a blood gas analyzer (BMS3 MK2; manufactured by Radiometer, Denmark). Moreover, its degree of oxygen saturation was measured with an oximeter ($OSM_2$ Hemoximeter; manufactured by Radiometer, Denmark).

Blood flow rate was measured according to the dye dilution method, using the measuring apparatus illustrated in Fig. 6. On the basis of a known theory (Steart's theory), blood flow rate is given by the following equation.

$$[\text{Blood flow rate}] = F_i(K_3 C_i - \overline{D}_m)/(\overline{D}_m - \overline{D}_r)$$

where $F_i$ is the injection rate of a dye solution, $K_3$ is a constant depending on the shape of the catheter and the extinction coefficient of the dye, $C_i$ is

the concentration of the dye solution, $\overline{D}_m$ is the difference between the transmittances of the two types of light having passed through the dye-containing blood during dye injection, and $\overline{D}_r$ is the difference between the transmittances of the two types of light before dye injection.

The blood sample contained in a reservoir 23 was allowed to flow through a silicone tube 25 under control with a screw clamp 24. A 2.5 mg/100 ml solution of Indocyanine Green (ICG) [Diano Green; manufactured by Daiichi Pharmaceutical Co., Ltd.] containing blood plasma was injected into silicone tube 25 through a 7F catheter (0.35 mm in diameter) having four outlets formed around it. A specially made motor-driven injection pump 27 was used to keep the injection rate of the ICG solution constant. Using a measuring catheter 28 in accordance with the present invention, measurements were made in the flow rate range of 90 to 600 ml/min by applying injection rates of 60 to 170 ml/min to a silicone tube having an inner diameter of 6.5 mm. Moreover, measurements were made in the flow rate range of 250 to 1,200 ml/min by applying injection rates of 70 to 240 ml/min to a silicone tube having an inner diameter of 10 mm.

The results obtained from the above-described measurements are given below.

[Measurement of the degree of oxygen saturation]

As previously described, if the hemoglobin concentration of the blood is constant, the relationship between the transmittance ratio of the two types of light and the degree of oxygen saturation is linear. That is, as shown in Fig. 7, the difference ($D_{670}$ - $D_{805}$) between a logarithmically converted value ($D_{670}$) of the transmittance at the measuring wavelength of 670 nm and a logrithmically converted value ($D_{805}$) of the transmittance at the reference wavelength of 805 nm gives linear plots against the degree of oxygen saturation, and their slope is proportional to the hemoglobin concentration. Accordingly, the degree of oxygen saturation can be calculated by determining the hemoglobin concentration according to some other technique.

In order to confirm that the measuring method of the present invention can eliminate measuring disturbances due to various factors existing within the blood vessel, the influences exerted by the inner wall of the blood vessel, the temperature of the blood, the pH of the blood, and the blood flow rate were examined.

When the catheter of the present invention touched the inner walls of blood vessels having different colors (white and black), the transmittances at the two wavelengths of 670 nm and 805 nm varied as shown in Fig. 8. However, the ratio between them remained unchanged, so that the noises were eliminated. When the temperature of the blood was varied between 22°C and 37°C, the transmittances remained unchanged. When the blood flow rate was increased, the transmittances at both wavelengths increased, but the ratio between them remained constant. When blood was sucked through the lumen, a turbulent flow was produced to cause pulse-like fluctuations in both the infrared light and the visible light. However, these fluctuations were eliminated in the ratio between their transmittances. Moreover, the influences of pH changes at 100% oxygen saturation are shown in Fig. 9. The transmittances at both wavelengths decreased with a rise in pH, but the ratio between them remained constant.

The relationship between the results of oxygen saturation measurement according to the method of the present invention and the results of measurement with a Hemoximeter is shown in Fig. 10. The coefficient of correlation was 0.99 (with a sample size of 63 and an oxygen saturation range of 13 to 100%) and the standard deviation was 1.1%.

[Measurement of blood flow rate]

In order to examine the relationship between ICG concentration and transmittance at 805 nm, the results of measurement are plotted in Fig. 11. This graph indicates that the linearity of the plots is maintained irrespective of hemoglobin concentration. Thus, the blood flow rate can be determined according to the above-described equation by monitoring the transmittances of the two types of light (i.e., the measuring light at 805 nm and the reference light) before dye injection and during dye injection.

The relationship between the results of blood flow rate measurement according to the method of the present invention and the results of direct measurement is shown in Fig. 12. The coefficient of correlation was 0.99 (with a sample size of 29 and a flow rate range of 90 to 1,172 ml/min) and the standard deviation was 1.7% (average flow rate = 480 ml/min)

## Claims

1. A measuring catheter having light-emitting and light-receiving optical fibers disposed within said catheter, the end faces of said light-emitting and light-receiving optical fibers being fixed to the outer sidewall of said catheter so that they are opposite to each other with a predetermined space therebetween.

**2.** A measuring catheter as claimed in claim 1 wherein the space between the end faces of said light-emitting and light-receiving optical fibers is in the range of 0.75 to 1.5 mm.

**3.** A measuring catheter as claimed in claim 1 wherein a marker substance transfer tube is disposed within said catheter and a plurality of marker substance outlets communicating with said tube are formed in the sidewall of said catheter.

**4.** A method for measuring the degree of oxygen saturation of blood which comprises providing a measuring catheter having light-emitting and light-receiving optical fibers disposed within said catheter, the end faces of said light-emitting and light-receiving optical fibers being fixed to the outer sidewall of said catheter so that they are opposite to each other with a predetermined space therebetween, supplying light having a wavelength of about 805 nm and visible light to said light-emitting optical fiber, either simultaneously or alternately, measuring the transmittances of said two types of light that are collected by said light-receiving optical fiber after having passed through the blood present in the gap between the end faces of both optical fibers, and determining the degree of oxygen saturation of the blood on the basis of the transmittances of said two types of light.

**5.** A method for measuring blood flow rate which comprises providing a measuring catheter having light-emitting and light-receiving optical fibers disposed within said catheter, the end faces of said light-emitting and light-receiving optical fibers being fixed to the outer sidewall of said catheter so that they are opposite to each other with a predetermined space therebetween, supplying light having a wavelength of about 805 nm and visible light or light having a wavelength of 900 nm or longer to said light-emitting optical fiber, either simultaneously or alternately, measuring the transmittances of said two types of light that are collected by said light-receiving optical fiber after having passed through the marker substance-containing blood present in the gap between the end faces of both optical fibers, and determining the blood flow rate on the basis of the transmittances of said two types of light.

**6.** A method as claimed in claim 5 wherein said intravascularly retained optical-fiber measuring catheter of transmission type is provided with a marker substance transfer tube disposed within said catheter and a plurality of marker substance outlets communicating with said tube are formed in the sidewall of said catheter, and wherein measurements are made while a marker substance is being discharged from said outlets.

FIG. 1

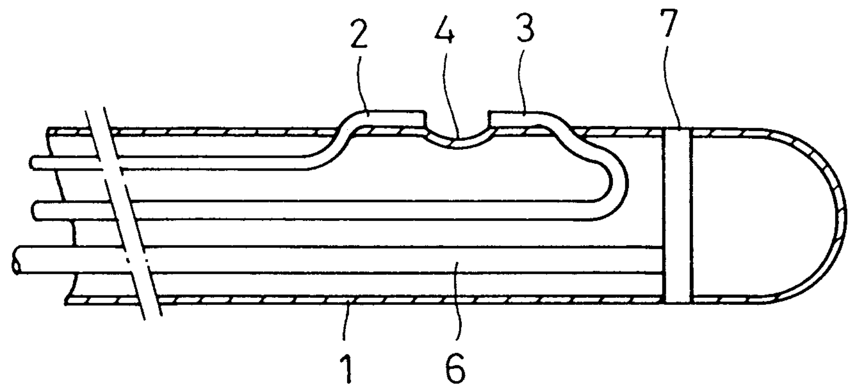


FIG. 2

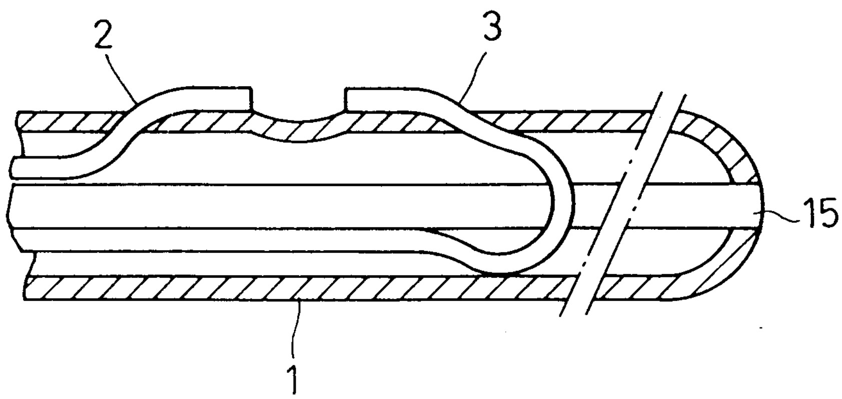

F I G. 3

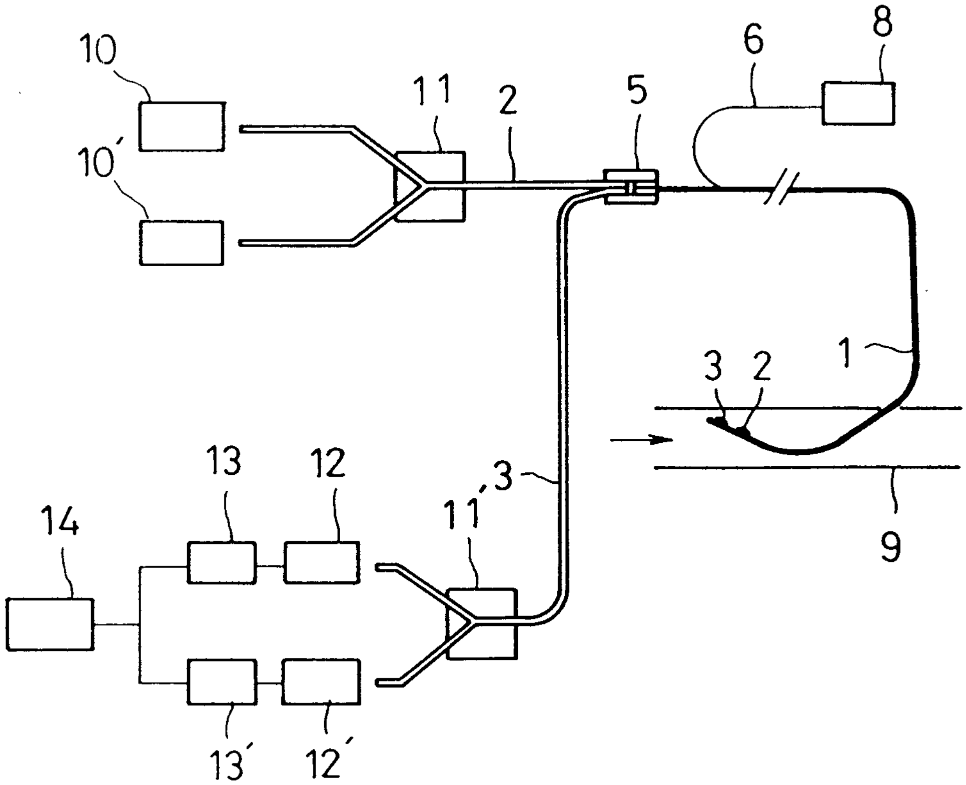

FIG. 4

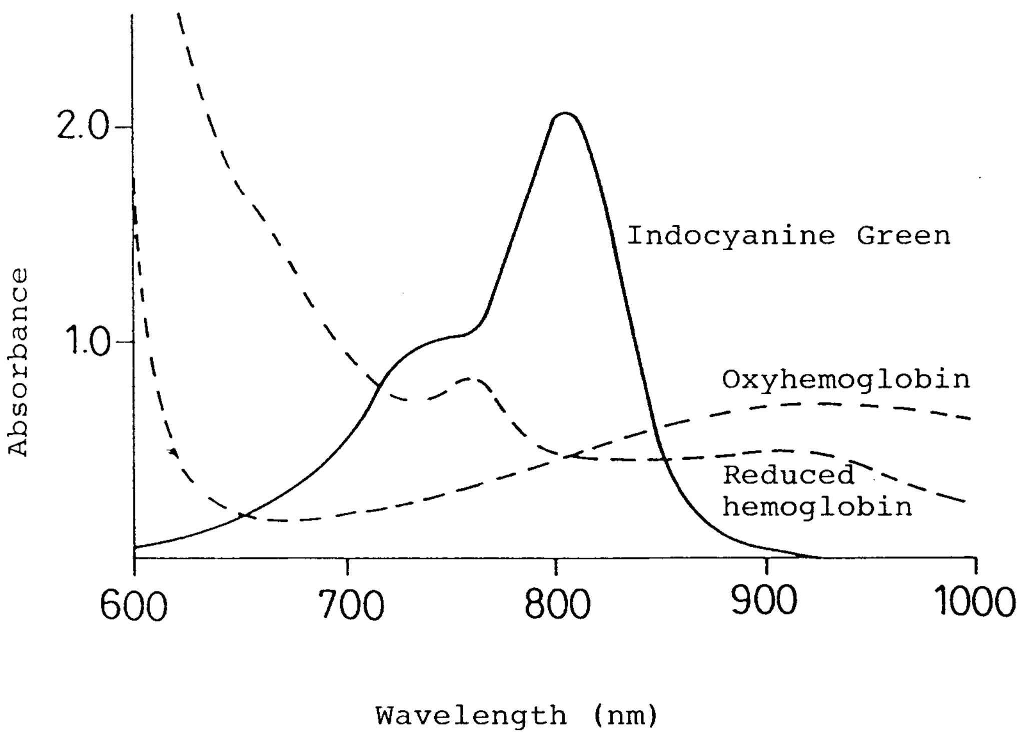
2.0
1.0
Absorbance
Indocyanine Green
Oxyhemoglobin
Reduced
hemoglobin
600
700
800
900
1000
Wavelength (nm)

FIG.5

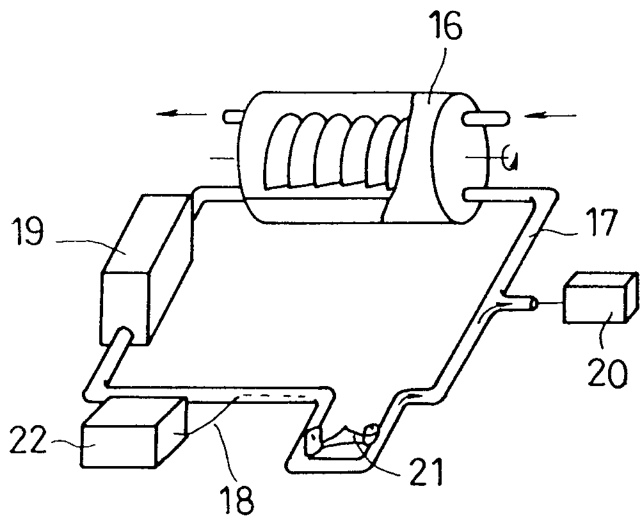


FIG.6

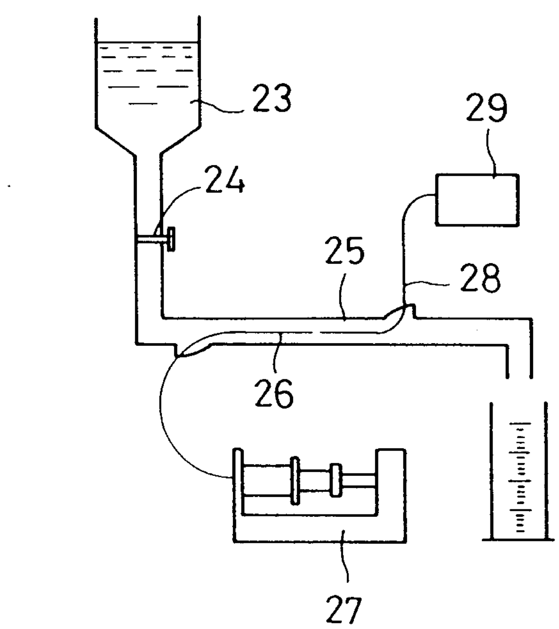

FIG. 7

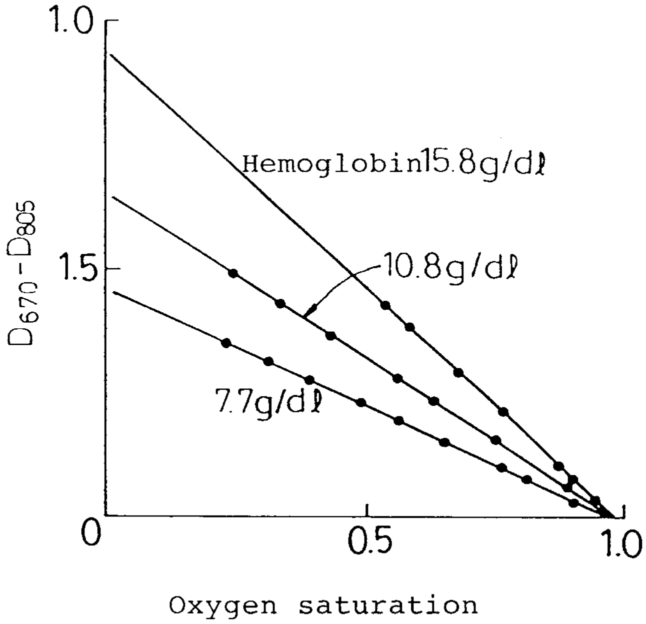
1.0
1.5
0
0.5
1.0
$D_{670}-D_{805}$
Hemoglobin15.8g/dℓ
10.8g/dℓ
7.7g/dℓ
Oxygen saturation

FIG. 8

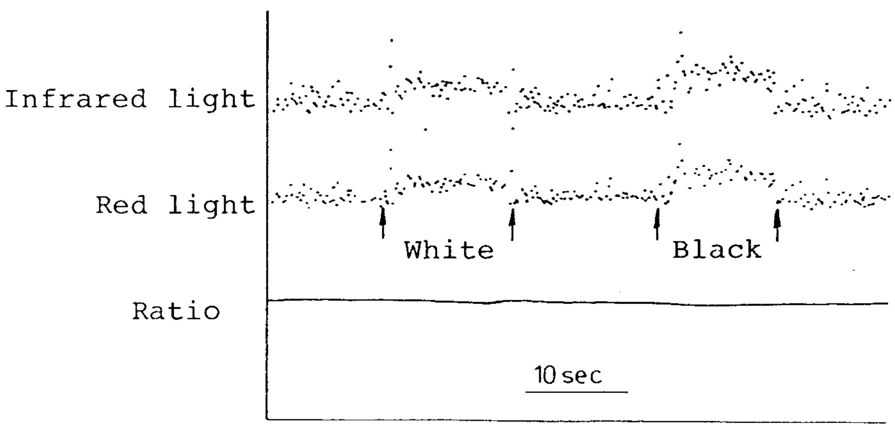
Infrared light
Red light
White
Black
Ratio
10 sec

FIG.9

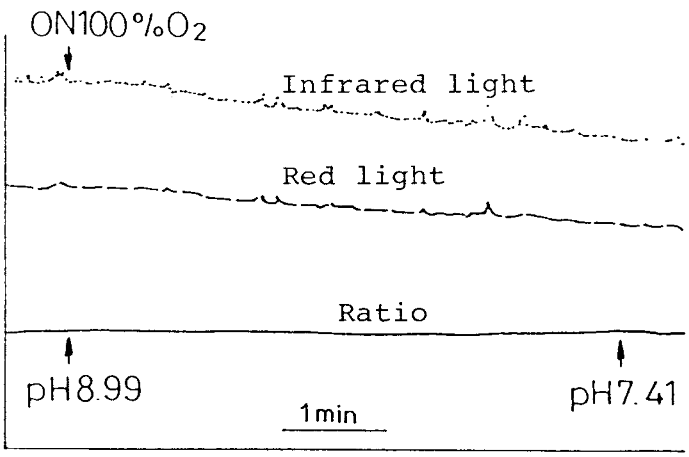
ON100%O2
Infrared light
Red light
Ratio
pH8.99
1min
pH7.41

FIG.10

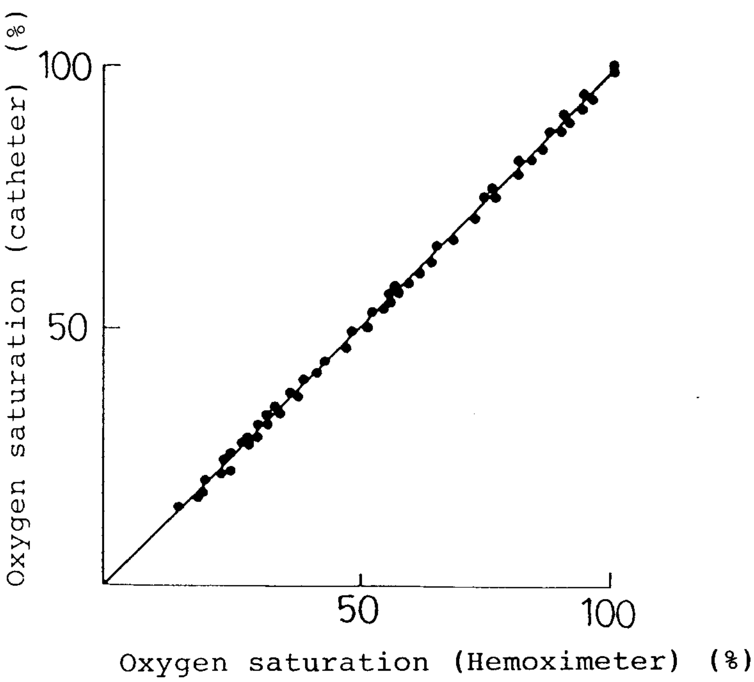
Oxygen saturation (catheter) (%)
100
50
50
100
Oxygen saturation (Hemoximeter) (%)

# F I G.11

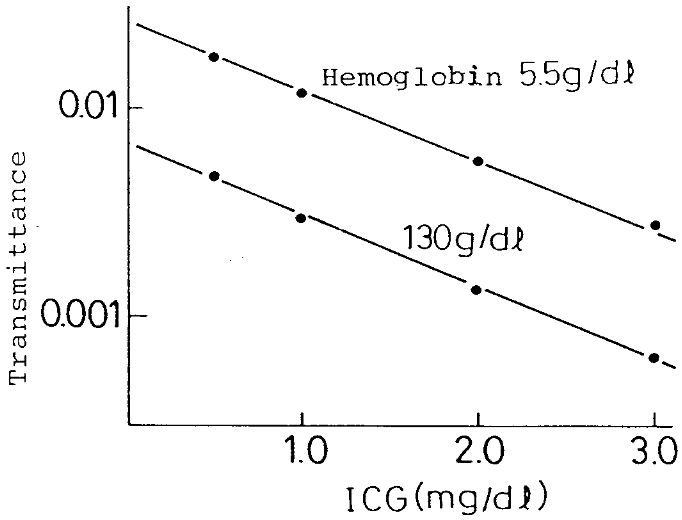
Hemoglobin 5.5g/dℓ
130g/dℓ
0.01
0.001
Transmittance
1.0
2.0
3.0
ICG(mg/dℓ)

# F I G.12

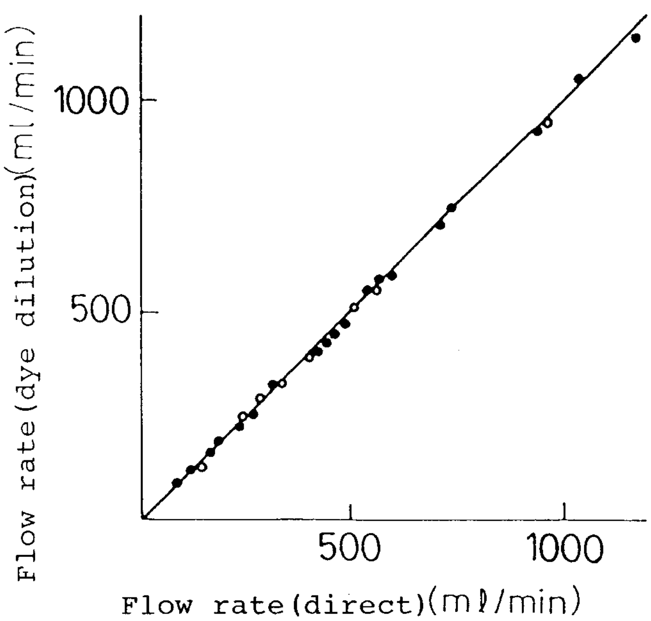
1000
500
Flow rate(dye dilution)(ml/min)
500
1000
Flow rate(direct)(mℓ/min)

# INTERNATIONAL SEARCH REPORT

International Application No PCT/JP91/00658

**I. CLASSIFICATION OF SUBJECT MATTER** (if several classification symbols apply, indicate all) [6]

According to International Patent Classification (IPC) or to both National Classification and IPC

Int. $Cl^5$ A61B5/02, A61B5/14

**II. FIELDS SEARCHED**

Minimum Documentation Searched [7]

| Classification System | Classification Symbols |
| --- | --- |
| IPC | A61B5/14, A61B5/02 |

Documentation Searched other than Minimum Documentation to the Extent that such Documents are Included in the Fields Searched [8]

| | |
| --- | --- |
| Jitsuyo Shinan Koho | 1955 - 1990 |
| Kokai Jitsuyo Shinan Koho | 1971 - 1991 |

**III. DOCUMENTS CONSIDERED TO BE RELEVANT** [9]

| Category * | Citation of Document, [11] with indication, where appropriate, of the relevant passages [12] | Relevant to Claim No. [13] |
| --- | --- | --- |
| Y | JP, U, 58-185203 (Sumitomo Electric Co., Ltd.), December 9, 1983 (09. 12. 83), (Family: none) | 1-6 |
| Y | JP, U, 47-35087 (Olympus Optical Co., Ltd.), December 19, 1972 (19. 12. 72), (Family: none) | 4 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
| --- | --- |
| July 23, 1991 (23. 07. 91) | August 12, 1991 (12. 08. 91) |
| International Searching Authority | Signature of Authorized Officer |
| Japanese Patent Office | |